# EUROPEAN PATENT SPECIFICATION

(11) **EP 0 821 940 B1**
(45) Date of publication and mention of the grant of the patent: **23.10.2002**
(21) Application number: 96830425.3
(22) Date of filing: 31.07.1996
(51) Int. Cl.: A61K 7/42

(54) **Sun screening preparations comprising triazine derivatives**
Triazinderivate enthaltendes Sonnenschutzmittel
Compositions photoprotectrices comprenant des dérivés de triazine

(43) Date of publication of application: 04.02.1998
(73) Proprietor: 3V SIGMA S.p.A, 20121 Milano (IT)
(72) Inventor: Malpede, Alverio, 24121 Bergamo (IT)
(74) Representative: Bianchetti, Giuseppe, Prof.

(56) References cited:
- EP-A- 0 507 692
- EP-A- 0 685 224
- US-A- 3 670 074
- US-A- 4 840 788
- US-A- 5 346 691
- US-A- 5 518 713

## Description

The present invention relates to cosmetic compositions (hereinafter called sun-protecting formulations) for the protection of skin and/or hair from ultraviolet radiations, containing new synergistic mixtures of sunscreens.

It is well known that sun radiation ranging from 280 to 400 nm is noxious for human skin; in particular that at wavelength ranging from 280 to 320 nm, the so called UV-B radiation, cause erythema and cutaneous burns, whose severity depends on duration of exposure and the kind of the skin.

It has been ascertained that also radiations ranging from 320 to 400 nm, so called UV-A and responsible of skin tanning can provoke noticeable alterations and damages to the skin, especially in cases of sensible skin or in case of continuous exposition to radiations.

A number of sun-protecting formulations were proposed for the photoprotection of skin and a very wide patent literature exists in this regard.

Very often these sun-protecting formulations are in the form of oil-in-water emulsion containing one or more organic lipophilic and/or hydrophilic sunscreens, in variable concentrations, capable of absorbing more or less intensely UV radiations of sun light.

The kind of sunscreens and their usable amount are selected depending on the desired sun protecting factor (SPF). SPF is an index of protection and is expressed as the ratio between the time of irradiation necessary to reach the erythematogenic threshold at the presence of the UV filter and the time necessary to reach the erythematogenic threshold in the absence of the UV filter.

It has now been found that the combination of particular sunscreens and precisely of one or more compounds of formula I, as described hereinafter, and of one or more derivatives of benzophenone of formula II, as described hereinafter, gives sun-protecting formulations an SPF higher than those obtainable by using, at the same concentrations, the compounds of formula I or those of formula II alone.

It is an object of the present invention a cosmetic composition, for the protection of human skin from sun radiation, comprising in admixture with vehicles and conventional excipients a combination of at least a compound of formula I 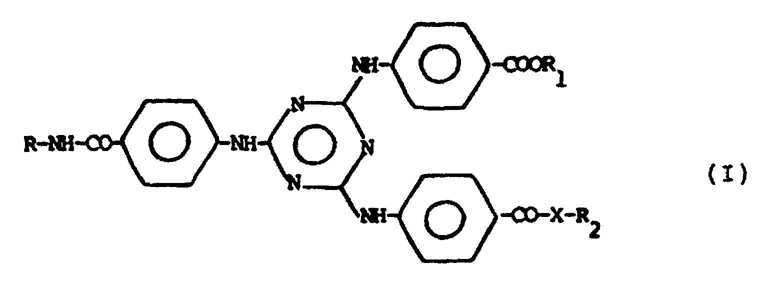 wherein R is C₁-C₈ straight or branched alkyl, C₅-C₁₂ cycloalkyl optionally substituted with one or more C₁-C₄ alkyl groups;
X is oxygen or the NH- group;
R₁ has the same meanings of R or is hydrogen, an alkali metal, an ammonium group or a group of formula III 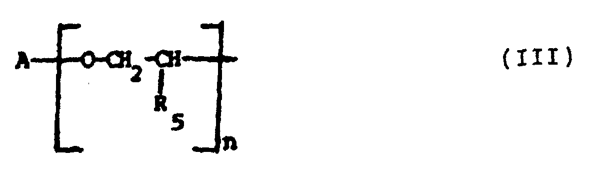 wherein A is C₁-C₈ straight or branched alkyl, C₅-C₈ cycloalkyl, aryl, which may be optionally substituted with one or more C₁-C₄ alkyls, R₅ is hydrogen or methyl and n can be an integer from 1 to 10;
R₂ has the same meanings of R when X is the group NH or has the same meaning of R₁ when X is oxygen;
with at least a compound of formula II 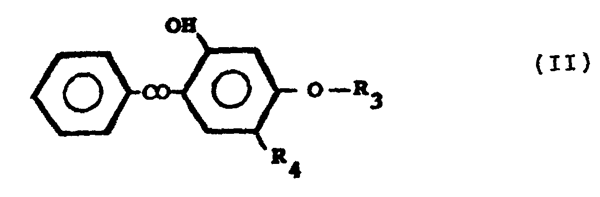 wherein
R₃ is hydrogen, methyl, n-octyl and R₄ is hydrogen, the SO₃H group or a salt thereof with an alkali metal or an amine.

Preferred compounds of formula I are those wherein:
- R= CH₃; R₁=R₂=C₄H₉-CH(C₂H₅)-CH₂-; X=O.
- R=(CH₃)₃C-CH₂-C(CH₃)₂-; R₁=R₂=C₄H₉-CH(C₂H₅)-CH₂-; X=O.
- R=R₂=(CH₃)₂CH; R₁=C₁₄H₂₉; X=NH.
- R=(CH₃)₃C-; R₁=R₂=C₄H₉-CH(C₂H₅)-CH₂-; X=O.

The compounds of formula I are described in US Patent 5346691, whereas the derivatives of benzophenone of formula II are well known and used since long time, and marketed by 3V-SIGMA under the trade name of Uvasorb 20H^{(R)}, UVASORB MET 3C^{(R)}, UVASORB S5^{(R)}.

According to the present invention, the sun-protecting formulations comprise a cosmetic substrate containing, with respect to the weight of the composition, from 1 to 15% of a derivative of benzophenone (compound of formula II) and from 0.5 to 10% of a compound of formula I.

The sun-protecting formulations according to -the present invention are applied on skin in order to protect it from the noxious action of the UV radiation, thus avoiding erythema or burns formation. Said compositions also serve for the treatment and then also for the protection of hair or of makeup in decorative cosmetics.

According to the present invention, in addition to the compounds of formula I and II, the cosmetic compositions can also contain one or more complementary hydrophilic or lipophilic sunscreens active in UV-B and/or in UV-A.

Examples of these other complementary sunscreens, usually used in cosmetics, are: derivatives of 4-methoxy-cinnamic acid, derivatives of salicylic acid, derivatives of benzylidenecamphor, derivatives of 2-phenyl-benzimidazole, derivatives of 2-cyano-3,3-diphenylacrylic acid, 3-(4-methylbenzylydene)-camphor; 3,3'-(1,4-phenylen-dimethylydene)bis(10-camphorsulfonic) acid; 2-ethylhexyl-2-cyano-3,3-diphenylacrylate; 2-ethylhexyl-4-methoxy cinnamate; menthylsalicylate; 2,4,6-trianilino-(p-carbo-2-ethylhexyloxy)-1,3,5-triazine; 2-phenylbenzimidazol-5-sulfonic acid; 4-methoxy-4-terbutyldibenzoylmethane; 4-isopropyldibenzoylmethane.

The sun-protecting formulations according to the present invention can also contain inorganic pigments usually used in cosmetics, such as for example titanium oxide, zinc oxide, silicon oxide or aluminium oxide.

According to the present invention the cosmetic compositions can be in the form of oil-in-water or water-in-oil emulsions, solutions, lotions, or can be also in the form of gels, lipsticks, aerosol.

The compositions of the present invention are prepared by formulating conventional ingredients, such as for example oils, fats, emulsifiers, hydrating agents, moisturizing agents, emollients, preservatives, surfactants, thickening agents, perfumes, pigments, dyes and other else, such as alcohols, polyols, electrolytes, siliconic derivatives. The more commonly used solvents are triglycerides of caprinic or caprilic acid, castor oil, esters of fatty acids with isopropanol, propylene glycol, glycerin, propylene glycol monomethyl- or monoethyl- or monobutyl ether.

The following examples further illustrate the invention:

### Example 1

3 sun-protecting formulations having the following composition were prepared:

| | |
|---|---|
| Triglycerides of fatty acid | 20.0 g |
| Cetylstearyl alcohol ethoxylated with 33 moles of ethylene oxide | 2.0 g |
| Cetylstearyl alcohol | 2.0 g |
| Lanoline | 4.0 g |
| Silicone oil | 0.4 g |
| Sunscreen | 5.0 g |
| Abiol (R) (Preservative by 3V-SIGMA) | 0.2 g |
| Synthalen (R) M (crosslinked polyacrylic acid by 3V-SIGMA) | 0.1 g |
| Triethanolamine | 0.15 g |
| Perfume | 0.3 g |
| Distilled water | q.s.to 100.0 |

The fatty phase was warmed to 80°C, sunscreen was added, then the mixture was added to water, which contained the hydrosoluble compounds, heated to 80°C. Stirring was maintained for 15-20 minutes. After slow cooling, perfume was added.
Sun-formulation n. 1: the sunscreen is the compound A of formula I wherein

R=(CH₃)₃C-CH₂-C(CH₃)₂-

R₁=R₂=C₄H₉-CH(C₂H₅)-CH₂; X=O

Sun-formulation n. 2: the sunscreen is 2-hydroxy-4-methoxy-benzophenone (Uvasorb MET ^{(R)} )
Sun-formulation n. 3: the sunscreen is a mixture of 3.5 g of compound A and 1.5 g of 2-hydroxy-4-methoxybenzophenone.

For each formulation sun protecting factor was determined according to the method described by B. Diffey and J. Robson in J. Soc. Cosmet. Chem. 40, 127-133 (1989).

The Table shows the results:

**TABLE**

| **Sun-formulation** | **SPF** |
|---|---|
| 1 | 6.2 |
| 2 | 3.7 |
| 3 | 7.5 |

### Example 2 - Lotion

**TABLE**

| | |
|---|---|
| Compound A | 2.5 g |
| 2-hydroxy-4-methoxy-benzophenone | 7.5 g |
| Octyl octanoate | 54.0 g |
| Triglycerides C₈-C₁₀ | 32.0 g |
| Dioctylcyclohexane | 10.0 g |
| Perfume | 0.1 g |

The mixture of the solvents was warmed to 60°C, under stirring, the two sunscreens were added, stirring was continued for 10-15 minutes, after cooling perfume was added.

### Example 3 - Oil/Water sun-cream

| | |
|---|---|
| C₁₂-C₁₅ alkyl benzoate | 5.0 g |
| Diisopropyl adipate | 5.0 g |
| Karitè Butter | 2.0 g |
| Bisabalol-A | 0.5 g |
| Compound B | 3.0 g |
| 2-hydroxy-4-methoxy-benzopheno-ne-5-sulphonic acid (Uvasorb S5) | 3.5 g |
| Stabylen 30 ^{(R)} (Thickening agent 3V-SIGMA) | 0.3 g |
| Synthalen K ^{(R)} (Thickening agent 3V-SIGMA) | 0.3 g |
| Abiol ^{(R)} (Preservative of the 3V-SIGMA) | 0.3 g |
| Methylparaben | 0.2 g |
| Propylparaben | 0.1 g |
| Glycerin | 5.0 g |
| Aminomethylpropanol | 0.5 g |
| Water | q.s. to 100.0 |
| Perfume | |

Compound B is the sunscreen of formula I wherein R=CH₃-; R₁=R₂=C₄H₉-CH(C₂H₅)-CH₂-; X=O

Fatty phase was warmed to 70°C, the sunscreens were added and stirring was continued for 10-15 minutes. Stabylen 30 and Synthalen K were dispersed in water and the resulting aqueous dispersion was heated to 70°C, the fatty phase was added under strong stirring. Neutralization with aminomethylpropanol followed, cooling to 35°C and preservatives, glycerin and perfume were added.

### Example 4 - Oil/water day-cream

| | |
|---|---|
| Triglyceryl methylglucose distearate | 4.0 g |
| Glyceryl stearate | 1.0 g |
| C₁₂-C₁₅ alkyl benzoate | 7.4 g |
| Avocado oil | 5.0 g |
| Diisopropyl adipate | 5.0 g |
| Compound C | 1.5 g |
| 2-hydroxy-4-methoxy-benzophenone | 3.5 g |
| Synthalen K (Thickening agent by 3V-SIGMA) | 0.2 g |
| Abiol (Preservative by 3V-SIGMA) | 0.3 g |
| Methylparaben | 0.2 g |
| Propylparaben | 0.1 g |
| Aminomethyl propanol | 0.15 g |
| Glycerin | 3.0 g |
| Distilled water | q.s. to 100.0 |

Compound C is the sunscreen of formula I wherein R=(CH₃)₃C-; R₁=R₂=C₄H₉-CH(C₂H₅)-CH₂-; X=O execution was as in Example 3.

### Example 5 - Water/oil sun-milk

| | |
|---|---|
| Hydrogenated castor oil ethoxylated with 7 moles of ethylene oxide | 7.5 g |
| Alcohols of lanoline in mineral oil | 2.5 g |
| Octyl octanoate | 7.5 g |
| Dioctylcyclohexane | 5.0 g |
| Cetylstearyl octanoate | 5.0 g |
| Compound B | 3.0 g |
| 2-hydroxy-4-methoxy-benzophenone | 5.0 g |
| Abiol (Preservative by 3V-SIGMA) | 0.3 g |
| Glycerin | 5.0 g |
| Water | q.s. to 100.0 |
| Perfume | |

The fatty phase was warmed to 70°C and sunscreens were added. Water, pre-heated to 70°C, was added to the fatty phase, under strong stirring. After cooling, preservative, glycerin and perfume were added.

### Example 6 - Lipstick

The base mixture consisting was first prepared:

| | |
|---|---|
| Beeswax | 13.0 g |
| Carnauba wax | 7.4 g |
| Lanoline | 5.0 g |
| Isopropyl myristate | 8.0 g |
| Mineral oil | 3.0 g |
| Castor oil | 63.5 g |

85 g of this mixture were heated to melt, to the molten mass 7 g of compound A and 7 g of 2-hydroxy-4-methoxy-benzophenone were added, as well as perfume and dyes, then the mixture was diluted to 1000 g with castor oil and cooled down to room temperature.

## Claims

1. Cosmetic compositions for the protection of human skin from sun radiation, comprising in admixture with conventional vehicles and excipients a combination of at least a compound of formula I 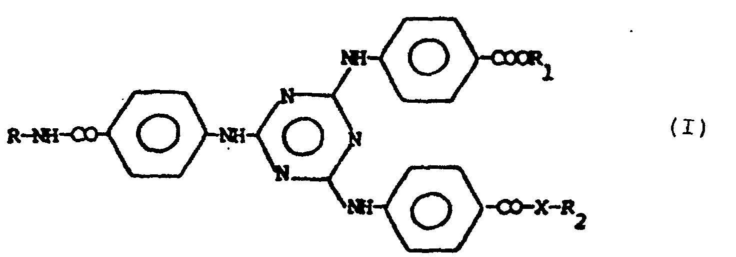 wherein R is C₁-C₈ straight or branched alkyl, C₅-C₁₂ cycloalkyl, optionally substituted with one or more C₁-C₄ alkyl groups;
X is oxygen or the NH- group;
R₁ has the same meanings of R or is hydrogen, an alkali metal, an ammonium group or a group of formula III 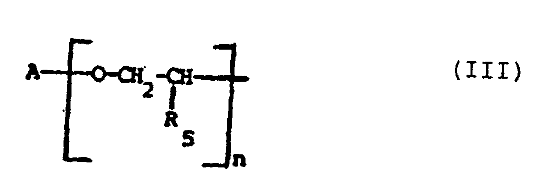 wherein A is C₁-C₈ straight or branched alkyl, C₅-C₈ cycloalkyl, aryl, optionally substituted with one or more C₁-C₄ alkyls, R₅ is hydrogen or methyl and n can be an integer from 1 to 10;
R₂ has the same meanings of R when X is the NH group or has the same meaning of R₁ when X is oxygen;
with at least a compound of formula II 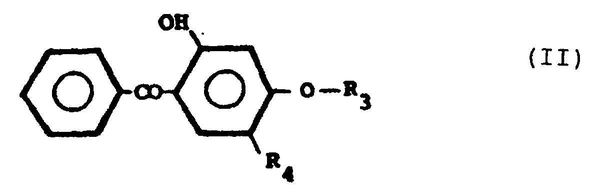 R₃ is hydrogen, methyl, n-octyl and R₄ is hydrogen, the SO₃H group or a salt thereof with an alkali metal or an amine.

2. Compositions according to claim 1, wherein in the compound of formula I X is oxygen.

3. Compositions according to claim 1, wherein in the compounds of formula I X is oxygen and R₁ and R₂ are C₁-C₈ straight or branched alkyl or C₆-C₁₂ cycloalkyl, optionally substituted with one or more C₁-C₄ alkyls.

4. Compositions according to claims 1-3 containing also zinc oxide.

5. Compositions according to claims 1-3 containing also titanium oxide.

6. Compositions according to claims 1-5 containing also other sunscreens selected from the group consisting of 3-(4-methylbenzylydene)-camphor; 3,3'-(1,4-phenylendimethylydene)bis(10-camphorsulfonic) acid; 2-ethylhexyl-2-cyano-3,3-diphenylacrylate; 2-ethylhexyl-4-methoxy-cinnamate; menthylsalicylate; 2,4,6-trianilino(p-carbo-2-ethylhexyloxy)-1,3,5-triazine; 2-phenylbenzimidazol-5-sulfonic acid; 4-methoxy-4-terbutyldibenzoylmethane; 4-isopropyldibenzoylmethane.

7. Compositions according to anyone of claims 1-6, containing as adjuvants one or more components selected from the group consisting of thickening agents, emollients, hydrating agents, preservatives, perfume.

8. Use of the compositions according to anyone of claims 1-7 for the cosmetic treatment of human skin or hair when exposed to sun radiation.

## Patentansprüche

1. Kosmetische Zusammensetzungen zum Schutz der menschlichen Haut vor Sonnenstrahlen, umfassend im Gemisch mit üblichen Vehikeln und Exzipienten eine Kombination von mindestens einer Verbindung der Formel I 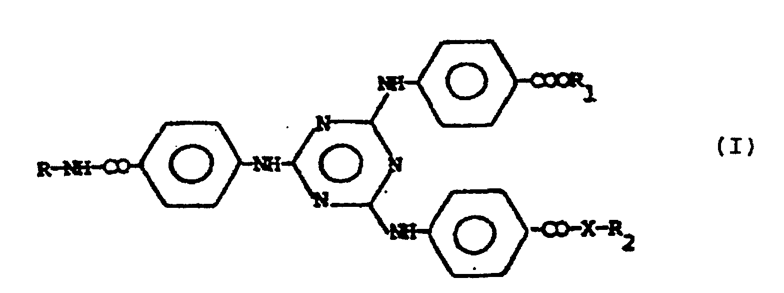 wobei R geradekettiges oder verzweigtes (C₁-C₈)Alkyl, (C₅-C₁₂)Cycloalkyl ist, gegebenenfalls substituiert mit einer oder mehr (C₁-C₄)Alkylgruppen;
X Sauerstoff oder eine NH-Gruppe ist;
R₁ dieselben Bedeutungen hat wie R oder Wasserstoff, ein Alkalimetall, eine Ammoniumgruppe oder eine Gruppe der Formel III ist 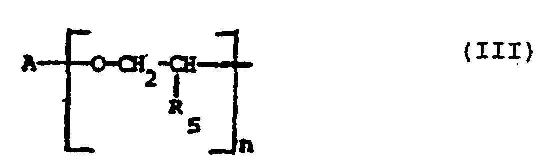 wobei A geradkettiges oder verzweigtes (C₁-C₈)Alkyl, (C₅-C₈)Cycloalkyl, Aryl ist, gegebenenfalls substituiert mit einem oder mehr (C₁-C₄)Alkyl, R₅ Wasserstoff oder Methyl ist und n eine ganze Zahl von 1 bis 10 sein kann;
R₂ dieselben Bedeutungen hat wie R, wenn X die NH-Gruppe ist, oder dieselbe Bedeutung hat wie R₁, wenn X Sauerstoff ist;
mit mindestens einer Verbindung der Formel II 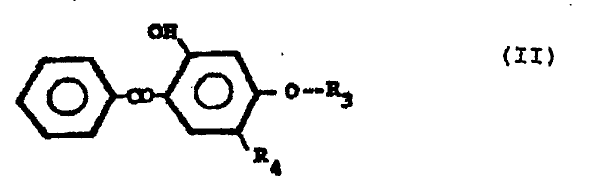 wobei R₃ Wasserstoff, Methyl, n-Octyl ist und R₄ Wasserstoff, eine SO₃H-Gruppe oder ein Salz davon mit.einem Alkalimetall oder einem Amin ist.

2. Zusammensetzungen nach Anspruch 1, wobei in der Verbindung der Formel I X Sauerstoff ist.

3. Zusammensetzungen nach Anspruch 1, wobei in den Verbindungen der Formel I X.Sauerstoff ist und R₁ und R₂ geradkettiges oder verzweigtes (C₁-C₈)Alkyl oder (C₆-C₁₂)Cycloalkyl sind, gegebenenfalls substituiert mit einem oder mehr (C₁-C₄)Alkyl.

4. Zusammensetzungen nach den Ansprüchen 1 bis 3, enthaltend auch Zinkoxid.

5. Zusammensetzungen nach den Ansprüchen 1 bis 3, enthaltend auch Titanoxid.

6. Zusammensetzungen nach den Ansprüchen 1 bis 5, enthaltend auch andere Sonnenschutzmittel, die ausgewählt werden aus der Gruppe, die besteht aus 3-(4-Methylbenzyliden)-kampfer; 3,3'-(1,4-Phenylen-dimethyliden)bis(10-kampfersulfon)säure; 2-Ethylhexyl-2-cyano-3,3-diphenylacrylat; 2-Ethylhexyl-4-methoxy-cinnamat; Menthylsalicylat; 2,4,6-Trianilino-(p-carbo-2-ethylhexyloxy)-1,3,5-triazin; 2-Phenylbenzimidazol-5-sulfonsäure; 4-Methoxy-4-tert-butyldibenzoylmethan; 4-Isopropyldibenzoylmethan.

7. Zusammensetzungen nach einem der Ansprüche 1 bis 6, enthaltend als Hilfsstoffe eine oder mehr Komponenten, die ausgewählt werden aus der aus Verdickungsmitteln, Weichmachern, Hydratisierungsmitteln, Konservierungsmitteln, Parfüm bestehenden Gruppe.

8. Verwendung der Zusammensetzungen nach einem der Ansprüche 1 bis 7 zur kosmetischen Behandlung der menschlichen Haut oder des menschlichen Haares bei Exposition gegen Sonnenbestrahlung.

## Revendications

1. Compositions cosmétiques pour la protection de la peau humaine contre le rayonnement solaire, comprenant, en mélange avec des véhicules et excipients conventionnels, une combinaison d'au moins un composé de formule I : 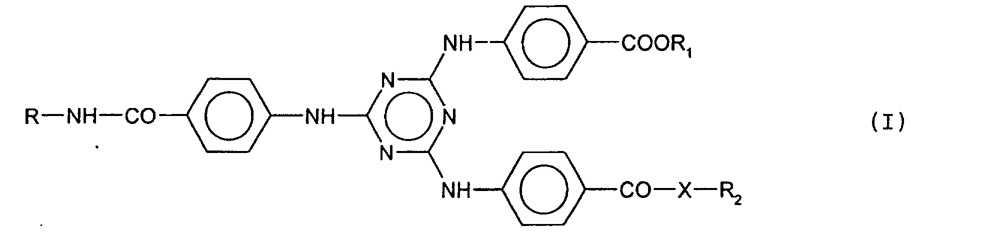 dans laquelle R est alkyle en C₁ à C₈ linéaire ou ramifié, cycloalkyle en C₅ à C₁₂, éventuellement substitué par un ou plusieurs groupes alkyle en C₁ à C₄ ;
X est oxygène ou le groupe NH- ;
R₁ a les mêmes significations que R ou est hydrogène, un métal alcalin, un groupe ammonium ou un groupe de formule III : 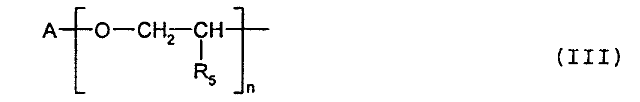 dans laquelle A est alkyle en C₁ à C₈ linéaire ou ramifié, cycloalkyle en C₅ à C₈, aryle, éventuellement substitué par un ou plusieurs groupes alkyle en C₁ à C₄, R₅ est hydrogène ou méthyle et n peut être un entier de 1 à 10 ;
R₂ a les mêmes significations que R quand X est le groupe NH ou a les mêmes significations que R₁ quand X est oxygène ;
avec au moins un composé de formule II : 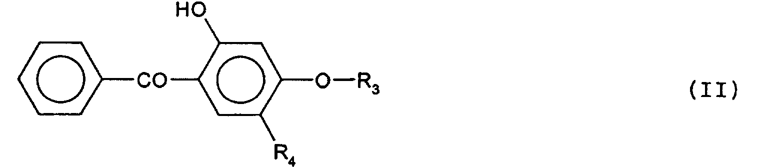 où R₃ est hydrogène, méthyle ou n-octyle et R₄ est hydrogène, le groupe SO₃H ou un de ses sels avec un métal alcalin ou une aminé.

2. Compositions selon la revendication 1, dans lesquelles, dans le composé de formule I, X est oxygène.

3. Compositions selon la revendication 1, dans lesquelles, dans les composés de formule I, X est oxygène et R₁ et R₂ sont alkyle en C₁ à C₈ linéaire ou ramifié ou cycloalkyle en C₆ à C₁₂, éventuellement substitués par un ou plusieurs alkyles en C₁ à C₄.

4. Compositions selon les revendications 1 à 3, contenant aussi de l'oxyde de zinc.

5. Compositions selon les revendications 1 à 3, contenant aussi de l'oxyde de titane.

6. Compositions selon les revendications 1 à 5, contenant aussi d'autres écrans solaires choisis dans l'ensemble constitué par le 3-(4-méthylbenzylidène)-camphre ; l'acide 3,3'-(1,1-phénylènediméthylidène)bis(10-camphosulfonique) ; le 2-cyano-3,3-diphénylacrylate de 2-éthylhexyle ; le 4-méthoxycinnamate de 2-éthylhexyle ; le salicylate de menthyle ; la 2,4,6-trianilino(p-carbo-2-éthylhexyloxy)-1,3,5-triazine ; l'acide 2-phénylbenzimidazole-5-sulfonique ; le 4-méthoxy-4-tert-butyldibenzoylméthane ; le 4-isopropyldibenzoylméthane.

7. Compositions selon l'une quelconque des revendications 1 à 6, contenant, à titre d'adjuvants, un ou plusieurs composants choisis dans l'ensemble constitué par les agents épaississants, les émollients, les agents hydratants, les conservateurs, les parfums.

8. Utilisation des compositions selon l'une quelconque des revendications 1 à 7 pour le traitement cosmétique de la peau ou des cheveux humains quand ils sont exposés au rayonnement solaire.
